**Europäisches Patentamt**

**European Patent Office**

**Office européen des brevets**

(19)

(11) Veröffentlichungsnummer : **0 051 236**
**B2**

(12)

# NEUE EUROPÄISCHE PATENTSCHRIFT

(45) Veröffentlichungstag der neuen Patentschrift :
09.11.88

(51) Int. Cl.⁴ : **C 07 C 19/06, C 07 C 17/33**

(21) Anmeldenummer : 81108877.2

(22) Anmeldetag : 24.10.81

(54) **Verfahren zur Herstellung von Tetrachlorkohlenstoff.**

(30) Priorität : 30.10.80 DE 3040851

(43) Veröffentlichungstag der Anmeldung :
12.05.82 Patentblatt 82/19

(45) Bekanntmachung des Hinweises auf die Patenterteilung : 12.12.84 Patentblatt 84/50

(45) Bekanntmachung des Hinweises auf die Entscheidung
über den Einspruch : 09.11.88 Patentblatt 88/45

(84) Benannte Vertragsstaaten :
DE

(56) Entgegenhaltungen :
DE-B- 1 618 531
DE-B- 2 150 400
DE-B- 2 231 049
DE-C- 1 074 025
US-A- 2 727 076
ULLMANNS ENCYKLOPÄDIE DER TECHNISCHEN
CHEMIE, 4. Auflage, Band 9, 1975, Seiten 461-463
VERLAG CHEMIE
Jap. Patentbekanntmachung Sho-49-16402

(73) Patentinhaber : HOECHST AKTIENGESELLSCHAFT
Postfach 80 03 20
D-6230 Frankfurt am Main 80 (DE)

(72) Erfinder : Rebhan, Dieter, Dr.
Kurhausstrasse 20B
D-6238 Hofheim am Taunus (DE)
Erfinder : Schmitz, Heinz, Dr.
Hörselbergstrasse 3
D-6230 Frankfurt am Main 80 (DE)

Jouve, 18, rue St-Denis, 75001 Paris, France

## Beschreibung

Es ist aus einer Reihe von Patentschriften (US-PS 3 651 157, GB-PS 1 311 501, US-PS 3 845 148) bekannt, Tetrachlorkohlenstoff durch Umsetzung von aliphatischen, cycloaliphatischen oder aromatischen Kohlenwasserstoffen, deren Chlorsubstitutionsprodukten oder Gemischen dieser Verbindungen mit Chlor in Abwesenheit von Katalysatoren in der Gasphase bei Drücken von 20 bis 800 bar herzustellen.

Bei dieser sogenannten Chlorolyse-Reaktion sollen die Einsatzmaterialien zunächst eine auf 8-400 °C gehaltene Vorreaktionszone durchlaufen und danach in einer Hauptreaktionszone bei 400-800 °C zu Tetrachlorkohlenstoff, sowie bei Anwesenheit wasserstoffhaltiger Verbindungen auch zu Chlorwasserstoff umgesetzt werden. Die Reaktion wird im allgemeinen in einem rohrförmigen Reaktor durchgeführt, der mit einer Nickelauskleidung versehen ist. Das Chlor wird vorzugsweise in einem Überschuß von 25 bis 300 % der stöchiometrischen Menge eingesetzt.

Bei der Durchführung der Chlorolyse können jedoch Störungen im Reaktionsteil vor allem Druckstöße, auftreten, die offenbar auf das Absinken der Temperatur in der Vorreaktionszone auf Werte unter ca. 250 °C, die Bildung einer flüssigen Phase und deren spontane Abreaktion zurückzuführen sind. Unter diesen Bedingungen ist keine stabile Fahrweise zu realisieren. Der Versuch, das genannte Absinken der Temperatur durch entsprechende Heizung des Reaktors sowie durch Erhöhung der Chlortemperatur und des Druckes zu verhindern, gelingt nicht.

Die vorliegende Erfindung löst die Aufgabe, das Absinken der Temperatur und die dadurch hervorgerufenen Störungen zu vermeiden.

Gegenstand der Erfindung ist ein Verfahren zur Herstellung von Tetrachlorkohlenstoff durch Umsetzung von aliphatischen, cycloaliphatischen oder aromatischen Kohlenwasserstoffen, deren Chlorsubstitutionsprodukten oder Gemischen dieser Verbindungen mit Chlor in der Gasphase bei Drücken von 20 bis 800 bar sowie bei Temperaturen von 400 bis 800 °C, dadurch gekennzeichnet, daß man die Reaktion in nur einer Reaktionszone durchführt und man die Einsatzmaterialien durch eine Düse in den Reaktor einleitet. Auf diese Weise werden die Einsatzmaterialien unmittelbar nach Eintritt in den Reaktor mit heißen Reaktionsprodukten vermischt. Die Düse ist eine Verengung des in den Reaktor hineinragenden Einleitrohres, durch die eine erhöhte Austrittsgeschwindigkeit der Einsatzmaterialien erreicht wird. Die Düse kann als Einstoff- oder als Zweistoff-Düse konstruiert sein. Eine besonders gute Vermischung wird durch zusätzliche Verwendung eines Leitrohres erreicht. Dieses an beiden Enden gegenüber dem Reaktorinnenraum offene Rohr wird in einer geringen Distanz von der Düse angebracht. Der in den Innenraum des Leitrohres mit hoher Auströmgeschwindigkeit eingedüste Strahl der Einsatzmaterialien bewirkt einen partiellen Produktumlauf um das Leitrohr, wobei heiße Reaktionsgase an der Außenseite entlang zur Eintrittsstelle der kalten Einsatzmaterialien gesaugt und dort mit diesen vermischt werden. Es sind jedoch auch andere Ausführungen der hier geschilderten Kombination von Düse und Leitrohr möglich.

Das Leitrohr ist im allgemeinen etwa zwei- bis zehnmal so lang wie der Innendurchmesser des Reaktors. Auch ohne Leitrohr bewirkt der aus der Düse austretende Strahl der Einsatzmaterialien eine in vielen Fällen ausreichende Umwälzung der Gase im ersten Teil des Reaktorrohres, so daß die Einsatzmaterialien sehr schnell das für den Start der Chlorolyse-Reaktion erforderliche relativ hohe Temperaturniveau erreichen. Ob eine Einstoff-Düse (mit einer Verengung am Ende des Einleitrohres oder mit einer Verengung des gesamten in den Reaktor hineinragenden Einleitrohres) oder eine Zweistoff-Düse, eine Düse mit Leitrohr oder eine andere Kombination mit Düse gewählt wird, hängt jedoch von den speziellen Versuchsbedingungen ab. In allen Fällen kommt es infolge der durch die Düse bedingten erhöhten Austrittsgeschwindigkeit des Einsatzgemisches aus dem Einleitrohr im ersten Teile des Reaktors zu einer intensiven Umwälzung.

Während bei einer Einstoff-Düse das organische Einsatzmaterial mit Chlor bereits vor dem Eintritt in den Reaktor vereinigt wird, erfolgt dies bei einer Zweistoff-Düse erst nach dem Austritt aus den beiden Düsenöffnungen, von denen beispielsweise die eine im Zentrum der Düse und die andere als konzentrischer Ringspalt angeordnet ist. Vorzugsweise verwendet man eine Zweistoff-Düse, insbesondere in Kombination mit einem Leitrohr.

Wie eingangs bereits erwähnt, wird die Chlorolyse von Kohlenwasserstoffen oder deren Chlorsubstitutionsprodukten üblicherweise in zwei Schritten durchgeführt, und zwar die Vorreaktion meist im ersten Drittel des Reaktors, die Hauptreaktion im restlichen Reaktorraum. Letzterer dient auch als Basis für die Berechnung der Raum-Zeit-Ausbeute, deren höchster Wert in der GB-PS 1 311 501 mit 5,06 kg Tetrachlorkohlenstoff pro Liter Reaktionsraum und Stunde angegeben ist. Da bei der vorliegenden Erfindung eine Vorreaktionszone entfällt, ist der Reaktor bei gleicher Leistung erheblich kürzer.

Ein weiterer Vorteil dieser Erfindung, neben der Verhinderung von Druckstößen und Rußbildung, besteht darin, daß auf die bisher im allgemeinen übliche Vorheizung der Reaktionspartner, vor allem des Chlors, in den meisten Fällen verzichtet werden kann. Sofern die vor allem eingesetzten Chlorkohlenwasserstoff-Gemische allerdings leicht kristallisierende Verbindungen wie Hexachlorethan (Fp = 186 °C) oder Hexachlorbenzol (Fp = 227 °C) enthalten, müssen sie auch weiterhin vorgeheizt werden. Eine Vorheizung kann auch dann erforderlich sein, wenn die Reaktionsenthalpie des organischen Einsatzmaterials selbst bei Reduzierung des Chlorüberschusses auf ca. 10 % nicht ausreicht, um die für die Spaltung der Kohlenstoff-Kohlenstoff-Bindung notwendige Temperatur von etwa 600 °C einzustellen. In jedem Falle reicht jedoch eine wesentlich schwächere Verheizung aus als bei der bisher üblichen Arbeitsweise mit Vorreaktionszone.

Durch den Einsatz kalten Chlors wird im Vergleich zur bisherigen Arbeitsweise

1. die für die Vorheizung benötigte Dampfmenge eingespart, und

2. diejenige Menge an Chlor erniedrigt, die über den stöchiometrischen Bedarf hinaus zur Begrenzung der maximalen Reaktorinnentemperatur auf den gewünschten Wert bei der vorzugsweise adiabatisch durchgeführten Reaktion notwendig ist.

Zur sicheren Vermeidung der Rußbildung im Reaktor und der damit verbundenen Störungen wird vorzugsweise ein Chlorüberschuß von 10 bis 150 % bezogen auf den stöchiometrischen Bedarf verwandt, gegenüber 25 bis 300 % bei den erwähnten bekannten Verfahren.

Die Verringerung der Chlorüberschußmenge bedeutet, daß bei gleicher $CCl_4$-Raum-Zeit-Leistung Chlorpumpen mit geringerer Förderleistung eingesetzt werden können. Weiterhin wird der gesamte Aufwand für die destillative Abtrennung des überschüssigen Chlors aus dem Produktgemisch reduziert, das im wesentlichen aus Tetrachlorkohlenstoff, Chlor, Chlorwasserstoff, sowie geringen Mengen an Hexachlorethan und Hexachlorbenzol besteht.

Durch die vorliegende Erfindung werden also sowohl der für die Herstellung einer bestimmten Menge Tetrachlorkohlenstoff erforderliche Reaktionsraum als auch die Chlorvorheiztemperatur erheblich reduziert. Kleinere Reaktoren bedeuten eine Verringerung der Investitionskosten, der Emission in Störfällen und des Aufwandes für die Notentspannung des Reaktorinhaltes in eine Natronlauge-Wäsche. Die Hauptvorteile geringerer Chlortemperaturen bestehen einerseits in der Reduzierung des Chlorüberschusses, des Energieverbrauchs sowohl für die Vorheizung der gesamten Chlormenge als auch für die destillative Abtrennung und Rückführung des überschüssigen Chlors, sowie in geringeren Investitionskosten für den Chlorkreislauf bei Neuanlagen. Schließlich wird durch die erfindungsgemäße Verfahrensweise ein gleichmäßiger Reaktionsablauf gewährleistet.

Die Umsetzung der Kohlenwasserstoffe und/ oder deren Chlorsubstitutionsprodukt mit Chlor wird bei Drücken von 20 bis 800 bar und Temperaturen von 400-800 °C durchgeführt. Der bevorzugte Druckbereich liegt zwischen 50 und 300 bar, die bevorzugte Reaktionstemperatur bei 500 bis 700 °C.

Einsatzmaterialien für das erfindungsgemäße Verfahren sind aliphatische, cycloaliphatische oder aromatische Kohlenwasserstoffe und/oder deren Chlorsubstitutionsprodukte. Im allgemeinen verwendet man aliphatische Verbindungen mit bis zu 20 C-Atomen, cycloaliphatische Verbindungen mit bis zu 8 C-Atomen oder aromatische Verbindungen mit bis zu 18 C-Atomen. Bevorzugt sind aliphatische Verbindungen mit bis zu 16, insbesondere bis zu 12 C-Atomen, cycloaliphatische Verbindungen mit bis zu 6 C-Atomen und aromatische Verbindungen mit bis zu 14, insbesondere bis zu 12 C-Atomen.

Vor allem sind geeignet :

als aliphatische Verbindungen Chlormethan, Dichlormethan, Trichlormethan, Chlorethan, Dichlorethan, Trichlorethan, Tetrachlorethan, Pentachlorethan, Hexachlorethan, Chlorethen, Dichlorethen, Trichlorethen, Tetrachlorethen, Chlorpropan, Dichlorpropan, Trichlorpropan, Propen, Chlorpropren, Dichlorpropen, Dichlorbutan, Trichlorbutan, Tetrachlorbutan, Hexachlorbutan, Chlorbuten, Dichlorbuten, Hexachlorbuten, Chlorbutadien, Hexachlorbutadien, Di- und Trimere des Chloroprens $(C_4H_5Cl)_2$ und $(C_4H_5Cl)_3$ ;

als cycloaliphatische Verbindungen Cyclopentadien, Hexachlorcyclopentadien, Octachlorcyclopenten, Dichlorcyclohexen, Hexachlorcyclohexan ;

als aromatische Verbindungen Benzol, Chlorbenzol, Dichlorbenzol, Trichlorbenzol, Tetrachlorbenzol, Pentachlorbenzol, Hexachlorbenzol, Toluol, Chlortoluol, Dichlortoluol, Benzylchlorid, Benzalchlorid, Naphthalin, Chlornaphthalin, Polychlornaphthalin, Chlordiphenyl, Dichlordiphenyl.

Der Reaktor hat einen drucktragenden Mantel aus warmfestem Stahl, der mit einer Nickelauskleidung versehen ist. Vorzugsweise liegt der Schwefelgehalt des Stahls unter 0,015 Gew.-% und der Kohlenstoffgehalt unter 0,08 Gew.-%. Die Nickelauskleidung hat vorzugsweise die folgenden Eigenschaften : Nickelgehalt mindestens 99 Gew.-%, Schwefelgehalt unter 0,01 Gew.-%, Kohlenstoffgehalt unter 0,02 Gew.-%, keine Schweißnähte oder Schweißpunkte. Außerdem empfiehlt es sich, dafür zu sorgen, daß die Oberflächen des Stahlmantels und der Nickelauskleidung beim Zusammenbau nicht durch Kohlenstoff oder Schwefel enthaltende Stoffe, wie etwa Maschinenöl, verunreinigt werden ; gegebenenfalls müssen solche Stoffe wieder sorgfältig abgewaschen werden.

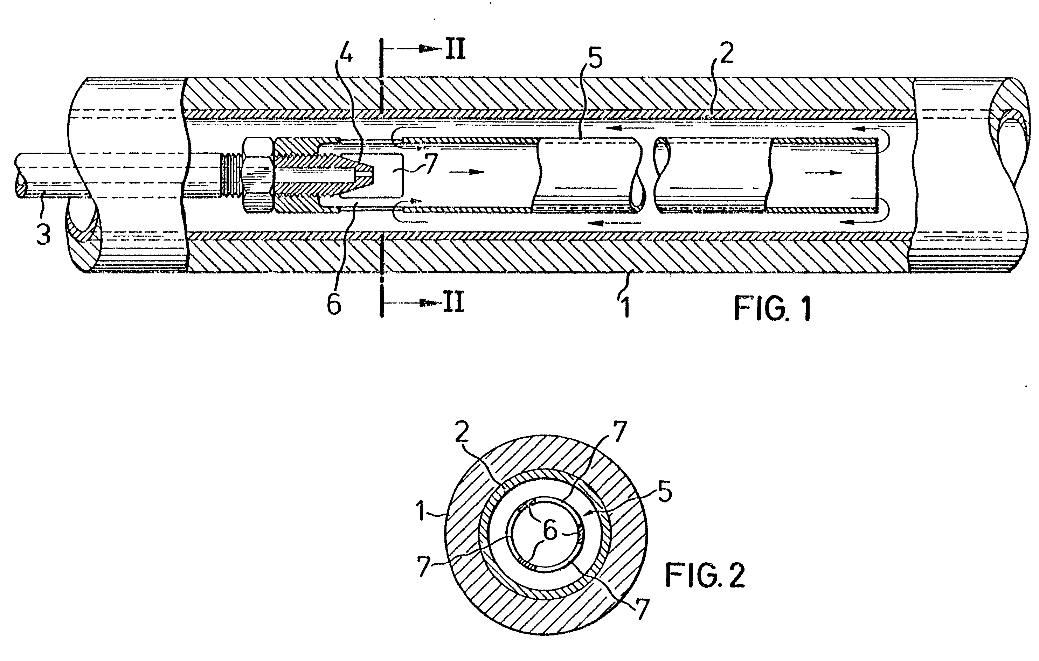

Fig. 1 zeigt einen Ausschnitt aus einem Reaktor mit Einstoff-Düse und Leitrohr. Die Reaktorwand (1) besteht aus warmfestem Stahl. Der Reaktor ist innen mit einer Nickelauskleidung (2) versehen. Das Gemisch aus Chlor und organischem Einsatzmaterial wird über Leitung (3) und Einstoff-Düse (4) in den Reaktor eingeleitet. Das Leitrohr (5) ist mit drei Stegen (6) an der Düse (4) befestigt. Durch die Zwischenräume (7) zwischen den Stegen (6) werden heiße Reaktionsgase angesaugt, die sich mit den aus der Düse (4) austretenden Einsatzmaterialien mischen.

Fig. 2 zeigt den Schnitt II-II von Fig. 1.

Die hier beschriebene Kombination einer Einstoff-Düse mit einem Leitrohr sowie die beschriebene konstruktive Ausführung dieser Kombination stellen jedoch nur eine von vielen Möglichkeiten des erfindungsgemäßen Verfahrens dar, die Vermischung der kalten Einsatzmaterialien mit heißen Reaktionsprodukten entscheidend zu verbessern.

Als « Länge » des Einleitrohrs wird in den folgenden Beispielen der Teil des Einleitrohrs bezeichnet, der in den Reaktor hineinragt.

Vergleichsbeispiel

Es wurde die Chlorolyse von 1,2-Dichlorethan in einem senkrechten Hochdruckrohr, versehen mit einer Nickelauskleidung, untersucht. Es hatte eine Länge von 2 200 mm, einen Außendurchmesser von 48 mm, eine lichte Weite von 25 mm und war an den Enden mittels Nickellinsen mit 4 mm-Bohrungen für die Produktein- und -ausgänge verschlossen. Der Reaktor wurde vor Beginn der Umsetzung mit vier Mantelheizungen auf 450-600 °C geheizt. Die maximale Reaktorinnentemperatur wurde durch Verschieben eines Thermoelements in einem mit Nickelrohr ummantelten Edelstahlrohr ermittelt, das von oben über eine Länge von 2 100 mm in den Reaktor hineinragte.

In diesem 1 l-Reaktor konnte die Umsetzung von 1,8 kg/h 1,2-Dichlorethan mit 15,0 kg/h ca. 150 °C heißem Chlor, das entspricht 232,8 % der stöchiometrischen Menge, bei 100 bar und einer maximalen Reaktionstemperatur von 670 °C nicht stationär durchgeführt werden, da sich die Zone der maximalen Temperatur trotz Zuheizung stetig zum Reaktorausgang verlagerte. Auch die Variation der Einsatzmengen, des Druckes und der Chlorvorheiztemperatur konnte diesen Nachteil auf Dauer nicht beseitigen.

Beispiel 1

Man geht vor wie im Vergleichsbeispiel, verändert man jedoch die Eintrittsstelle des Gemisches beider Reaktionspartner derart, daß man ein 100 mm langes Einleitrohr einbaut, dessen lichte Weite sich am Ende von 4 auf 1 mm verengt, und dieses mit einem Leitrohr (lichte Weite 15 mm, Länge 200 mm) versieht. Nunmehr gelingt die Einstellung einer stationären Fahrweise bei ausgeschalteten Reaktorheizungen.

Unter Einsatz von 16,2 kg/h 1,2-Dichlorethan und 77,2 kg/h nicht vorgeheiztem Chlor, das entspricht 133,1 % der stöchiometrischen Menge, wurde bei 150 bar sowie einer maximalen Reaktionstemperatur von 600 °C Tetrachlorkohlenstoff in einer Ausbeute von 98,8 % und einer Raum-Zeit-Ausbeute von 54,1 kg/l · h, bezogen auf das freie Reaktorvolumen von 0,92 l, hergestellt.

Beispiel 2

In der gleichen Apparatur wie in Beispiel 1 wurde auch ein Gemisch im wesentlichen aus (in Gewichtsprozenten) 1,2-Dichlorpropan (35,6 %), 1,2-Dichlorethan (17,8 %), Tetrachlorkohlenstoff (9,5 %), Hexachlorethan (9,3 %), 1,1,2-Trichlorethan (6,1 %), Dichlorbutenen (5,0 %), Trichlormethan (3,9 %), Tetrachlorethan (2,5 %), Chlorethan (2,4 %), Hexachlorbutadien (1,9 %), Chlorbenzol (1,0 %) und Pentachlorethan (1,0 %) bei 120 bar und 620 °C zu Tetrachlorkohlenstoff mit einer Ausbeute von 93,7 % und einer Raum-Zeit-Ausbeute von 58,4 kg/l · h chlorolysiert.

Beispiel 3

Der in Beispiel 1 beschriebene Versuch mit 1,2-Dichlorethan ließ sich auch. mit einem Einleitrohr von 50 mm Länge und/oder einem Leitrohr von 150 mm Länge sowie mit Düsen von 0,7 bzw. 2 mm lichter Weite mit im wesentlichen denselben Ergebnissen durchführen.

Mit Hilfe der Einstoffdüse und des Leitrohres war es also möglich, die Raum-Zeit-Leistungen gegenüber dem in der GB-PS 1 311 501 angegebenen höchsten Wert um mehr als das Zehnfache zu steigern. Selbst Chlorkohlenwasserstoffgemische mit doppelbindungshaltigen Komponenten ließen sich kalt in den über 400 °C heißen Umlauf um das konzentrische Leitrohr ohne Bildung störender Verkokungsprodukte eindosieren.

Beispiel 4

Es wurde die gleiche Chlorolyse-Apparatur wie in Beispiel 1 verwandt, jedoch mit dem Unterschied, daß anstelle des 100 mm langen Einleitrohres mit Düse und Leitrohr eine Zweistoffdüse (Länge 55 mm, Außendurchmesser 24 mm) eingebaut worden war. Diese bestand am Ende aus einer zentralen Bohrung mit einem Durchmesser von 1 mm sowie einem Ringspalt mit einem Außendurchmesser von 2,5 mm und einem Innendurchmesser von 2 mm.

Durch die zentrale Bohrung dieser Düse wurden 11,1 kg/h eines bei 100 bar kurz vorher hergestellten homogenen Gemisches aus 1,1 kg/h Cyclopentadien und 10,0 kg/h 1,2-Dichlorethan, durch den Ringspalt hingegen 70,7 kg/h kaltes, flüssiges Chlor, das entspricht 138,2 % des stöchiometrischen Bedarfs, eindosiert. Bei der wenige Zentimeter hinter dem Ende der Zweistoffdüse erreichten maximalen Reaktionstemperatur von 635 °C wurde Tetrachlorkohlenstoff in einer Ausbeute von 94,4 % und einer Raum-Zeit-Ausbeute von 46,0 kg/l · h, bezogen auf das freie Reaktionsvolumen von 0,90 l, erhalten.

Mit Hilfe der Zweistoffdüse konnten also in dem 1 l-Reaktor sowohl die getrennt eindosierten Reaktionspartner miteinander als auch mit den heißen Reaktionsprodukten intensiv vermischt werden. Dadurch konnte ebenfalls eine gleichmäßige störungsfreie Chlorolyse durchgeführt und hohe Ausbeuten sowie Raum-Zeit-Ausbeuten an Tetrachlorkohlenstoff erzielt werden.

**Patentansprüche**

1. Verfahren zur Herstellung von Tetrachlorkohlenstoff durch Umsetzung von aliphatischen, cycloaliphatischen oder aromatischen Kohlenwasserstoffen, deren Chlorsubstitutionsprodukten oder Gemischen dieser Verbindungen mit Chlor in der Gasphase bei Drücken von 20 bis 800 bar sowie bei Temperaturen von 400 bis 800 °C, dadurch gekennzeichnet, daß man die Reaktion in nur einer Reaktionszone durchführt und man die Einsatzmaterialien durch eine Düse in den Reaktor einleitet.

2. Verfahren nach Anspruch 1, dadurch gekennzeichnet, daß man eine Zweistoff-Düse verwendet.

3. Verfahren nach Anspruch 1 oder 2, dadurch gekennzeichnet, daß man im Reaktor zusätzlich zur Düse in geringer Distanz von dieser ein Leitrohr anbringt, das an beiden Enden offen gegenüber dem Reaktorinnenraum ist.

## Claims

1. Process for the preparation of carbon tetrachloride by reacting aliphatic, cycloaliphatic or aromatic hydrocarbon, chlorosubstitution products thereof or mixtures of these compounds with chlorine in the gaseous phase under a pressure of from 20 to 800 bar at a temperature of from 400 to 800 °C, with comprises carrying out the reaction in one reaction zone only and injecting the feed products into the reactor through a nozzle.

2. The process according to claim 1, which comprises using a binary nozzle.

3. The process according to claim 1 or 2, which comprises inserting in the reactor in addition to the nozzele a guide tube at a small distance to the nozzle, this tube being open at both ends with respect to the inner space of the reactor.

## Revendications

1. Procédé de fabrication de tétrachlorure de carbone par réaction d'hydrocarbures aliphatiques, cycloaliphatiques ou aromatiques, de produits de chlorosubstitution de ces hydrocarbures ou de mélanges de ces composés, avec du chlore, en phase gazeuse, sous une pression de 20 à 800 bar à une température de 400 à 800 °C, procédé caractérisé en ce qu'on effectue la réaction dans une seule zone de réaction et en ce qu'on injecte les produits devant participer à la réaction dans le réacteur par une buse.

2. Procédé selon la revendication 1, caractérisé en ce qu'on utilise une buse binaire.

3. Procédé selon la revendication 1 ou 2, caractérisé en ce qu'on agence dans le réacteur, en plus de la buse, à une faible distance de celle-ci, un tube de guidage ouvert à ses deux extrémités à l'égard du volume intérieur du réacteur.

FIG.1

FIG.2